Europäisches Patentamt

**European Patent Office**

**Office européen des brevets**

(11) Numéro de publication:

**0 006 376**
A1

## (12) DEMANDE DE BREVET EUROPEEN

(21) Numéro de dépôt: 79400360.8

(22) Date de dépôt: 05.06.79

(51) Int. Cl.³: **G 01 N 33/18, C 02 F 3/00**

(30) Priorité: 15.06.78 FR 7818489

(43) Date de publication de la demande: 09.01.80
Bulletin 80/1

(84) Etats contractants désignés: BE CH DE FR GB IT NL SE

(71) Demandeur: RHONE POULENC INDUSTRIES,
22, avenue Montaigne, F-75008 - Paris (FR)

(72) Inventeur: Herschke, Béatrice, 35, Chemin des Petites
Brosses, F-69300 - Caluire (FR)

(74) Mandataire: Cazes, Jean-Marie et al, RHONE
POULENC Service Brevets Chimie et Polymères
B.P. 753, F-75360 Paris Cedex 08 (FR)

(54) Procédé de mesure et/ou de détection de toxicité et appareillage pour ledit procédé.

(57) L'Invention concerne un procédé de mesure et/ou
de détection de la toxicité de liquides aqueux et un appareillage pour ledit procédé.

Selon ce procédé on procède à une culture de bactéries aérobies de manière à avoir une suspension aqueuse bactérienne (21) à caractéristiques stables, une fraction de cette culture de bactéries est mélangée au liquide aqueux (9) à tester préalablement saturé en gaz
oxygéné et le mélange est introduit dans un réacteur
piston (11), puis on mesure la quantité d'oxygène présente dans le liquide à la sortie de ce réacteur piston
(11) avec la sonde à oxygène (12).

TITRE MODIFIÉ
voir page de garde

## PROCEDE DE MESURE ET/OU DE DETECTION DE TOXICITE

La présente invention concerne un procédé de détection et/ou de mesure de la toxicité de liquides aqueux.

On sait que la détection et la mesure de la toxicité des liquides aqueux est un problème important notamment en ce qui concerne les eaux résiduaires, les effluents mais aussi les eaux de rivières, les eaux alimentant les nappes phréatiques, les eaux de puits, les eaux de nappes phréatiques, les eaux d'ultrafiltration, et les eaux de ruissellement et en général toutes les eaux arrivant soit à une station d'épuration biologique des eaux soit à une source d'alimentation en eau potable d'une communauté ou d'une localité.

Bien des qualités sont requises des procédés de détection et de mesures de toxicités. Il est d'abord souhaitable que la mesure puisse être faite en continu ; il est ensuite souhaitable que la mesure soit rapide, sensible et nette ; autrement dit, il est souhaitable qu'à l'arrivée d'une toxicité quelconque, le procédé et l'appareil de mesure de toxicité donnent le plus rapidement possible un signal important, net et précis.

Divers systèmes ont déjà été proposés. Ainsi dans le brevet français 2 228 407 on alimente une cellule de mesure simultanément par une culture bactérienne et l'eau à analyser. Ce système ne donne cependant pas de réponse assez fine, importante, précise et rapide. Dans le brevet 2 266 885, on met en contact l'eau à analyser avec un lit biologique submergé puis on procède à la mesure de la teneur résiduelle en oxygène du liquide quittant le lit. Mais ce système a l'inconvénient que lorsque les bactéries sont tuées par l'arrivée d'une première toxicité, il n'est pas possible de détecter une seconde toxicité avant que l'on n'ait préalablement régénéré le lit biologique submergé.

Un but de l'invention est de remédier aux inconvénients des procédés connus.

Un autre but de l'invention est de fournir un procédé simple continu, sensible et rapide de détection de la toxicité des liquides aqueux.

Un autre but de l'invention est de fournir un appareil permettant de mettre en oeuvre le procédé de l'invention.

Le procédé de l'invention est caractérisé en ce que l'on procède à une culture de bactéries aérobies de manière à avoir une suspension aqueuse bactérienne à caractéristiques stables, qu'une fraction de cette culture de bactéries est mélangée au liquide aqueux à tester préalablement saturé en gaz oxygéné et le mélange est introduit dans un réacteur piston, puis l'on mesure la quantité d'oxygène présent dans le liquide à la sortie de ce réacteur piston.

Toutes ces opérations peuvent être effectuées et sont préférentiellement effectuées en continu.

Le réacteur piston (connu en anglais sous le nom plug-flow reactor) utilisé dans l'invention est le plus souvent et le plus commodément un tube long et mince ; ce tube peut être rectiligne ou avoir les formes les plus diverses ; il peut être replié sur lui-même ou plus simplement enroulé, par exemple en hélice, spirale, en serpentin. Généralement, le rapport longueur/diamètre de ce tube est supérieur à 300, de préférence compris entre 5000 et 50 000. En outre le débit dans ce tube ainsi que le diamètre et la longueur du tube sont généralement tels que le temps de séjour du mélange dans le tube est compris entre 30 secondes et 15 minutes, de préférence entre 1 et 5 minutes.

Le temps de séjour du mélange (liquide aqueux à tester + fraction de culture bactérienne) avant le réacteur piston est le plus réduit possible et selon la modalité ordinaire et la plus avantageuse de l'invention, il n'y a pas de temps de séjour du mélange avant le réacteur piston ; lorsque le mélange est effectué dans une simple tubulure, celle-ci est considérée comme faisant partie du réacteur piston.

Le gaz oxygéné saturant le liquide aqueux à tester est généralement de l'air ou de l'air enrichi en oxygène ou de l'oxygène ; préférentiellement il s'agit d'air ; on peut aussi utiliser tout mélange air + azote ou azote + oxygène.

Selon une caractéristique avantageuse et préférentielle, la surface intérieure du réacteur piston est lisse. Dans le but de maintenir

.../...

cette surface à l'état lisse, on procède avantageusement à un nettoyage périodique par injection périodique d'un agent nettoyant dans le réacteur piston. La périodicité de cette injection est généralement comprise entre 10 mn et 1 heure ; l'agent nettoyant est de préférence biocide et acide ; les acides forts, minéraux ou organiques sont les agents de nettoyage préférés. L'injection d'un agent biocide et acide est particulèrement intéressante, car en dehors de l'effet nettoyant, cette injection permet un contrôle très régulier de la bonne marche de l'installation ce qui donne une garantie de sécurité et de fiabilité très grande ; en outre cela permet un certain étalonnage de la réponse de l'appareillage à l'arrivée d'agents toxiques. Selon une variante particulière de l'invention, l'injection de l'agent nettoyant est effectuée en associant un agent de neutralisation partiel de cet agent, de manière à ce que le pH du mélange culture bactérienne + liquide aqueux à tester soit compris entre 0 et 3.

La mesure de la quantité d'oxygène dans le liquide se fait par tout moyen connu en soi mais de préférence à l'aide d'une sonde à oxygène, par exemple une électrode à oxygène. Une telle sonde mesure en réalité une teneur en oxygène dissous mais, par étalonnage adéquat, elle peut servir à mesurer une teneur en gaz oxygène. Cette sonde plonge donc dans une cellule de mesure à laquelle aboutit le réacteur piston ; le volume de cette cellule de mesure est le plus petit possible ; il est généralement inférieur à 10 cm3, de préférence inférieur à 5 cm3 ; pour des questions d'ordre purement technologique, ce volume est généralement supérieur à 0,05 cm3. Le liquide contenu dans cette cellule de mesure est soutiré mais n'est pas réinjecté dans la culture bactérienne initiale ce qui fait que le procédé de l'invention utilise toujours une culture neuve. Par ailleurs, on procède de manière que le contenu de la cellule de mesure soit homogène, ce qui peut notamment être obtenu soit simplement par l'utilisation d'une cellule de mesure de très petit volume, soit lorsque la cellule de mesure a un volume plus grand, par des moyens d'agitation du contenu de cette cellule de mesure.

Pour obtenir une détection plus nette et plus claire de toxicité de l'effluent, il est préférable de compléter le mélange culture bactérienne + liquide aqueux à tester avec un nutrient. Ce nutrient peut être simplement introduit à l'entrée du réacteur piston ; il peut aussi être mélangé au liquide aqueux à tester peu avant son introduction dans le réacteur ou à la fraction de culture bactérienne peu avant son introduction dans le réacteur piston.                    .../...

- 4 -

0006376

Comme nutrient on utilise généralement des substrats assimilables par les bactéries. Comme substrats les plus commodes on utilise généralement les oses simples ou les oligosaccharides ou les polysaccharides ou les acides aminés ou les peptides. Les acides organiques ou leurs sels sont aussi utilisables. Plus généralement, on peut utiliser tous les composés (surtout organiques) assimilables par les bactéries et stimulant leur métabolisme. Bien entendu ces composés peuvent être utilisés seuls ou en mélange. Parmi les composés plus spécifiquement utilisables comme nutrient, on peut citer le glucose, le lactose, le saccharose, l'éthanol, les peptones ; les peptones peuvent notamment provenir d'hydrolyses acides, alcalines, pepsiques, pancréatiques, papaïnique ou par le moyen d'enzymes autolytiques, ces hydrolyses intervenant sur des substrats tels que caséine, gélatine, lactalbumine, soja, lysats de levures, tissus animaux notamment le coeur.

Le liquide aqueux à tester, préalablement à la mesure de la toxicité, est saturé en gaz oxygéné, notamment en air ou en air enrichi en oxygène ou en oxygène pur ou plus généralement par tout mélange oxygène + gaz inerte. Sur un plan pratique, il est plus commode de procéder à une saturation par l'air ; cette saturation est généralement effectuée par barbotage mais tout autre moyen d'introduction de gaz dans un liquide peut être utilisé (injection, éjection, diffusion à travers une membrane etc...)

La culture bactérienne aérobie utilisée pour l'invention peut être de tout type connu. Il s'agit d'une culture sous forme de suspension aqueuse et non de lit bactérien. Il est désirable, pour obtenir des résultats réguliers, d'avoir une culture bactérienne stable tant quant à sa nature que quant à l'état des bactéries (par stable on entend qu'il n'y a pas de variation substantielle des caractéristiques sur un espace de temps de quelques heures, 5 par exemple). Un moyen commode d'obtenir une culture bactérienne est de laisser se développer les bactéries aérobies d'un milieu quelconque en les alimentant en continu par un milieu nutritif donné. Il se produit ainsi un enrichissement progressif en bactéries les plus adaptées à ce milieu. Au bout de quelques temps (2 à 3 semaines par exemple) on dispose d'une population bactérienne stable et parfaitement adaptée à son milieu. Si l'on veut que le système selon l'invention ne détecte pas certains types de toxicité (par exemple dans le cas où certains polluants majeurs sont connus et leur arrivée est

.../...

également connue), on peut habituer la souche bactérienne à se développer en présence de ces polluants particuliers ce qui aura, pour effet qu'elle ne les détectera plus, tout au moins aux doses égales ou inférieures à la dose à laquelle elle est habituée.

Les conditions de culture de la culture bactérienne et notamment la quantité et la nature de l'agent nutritif sont habituellement choisis de manière que la teneur en gaz oxygéné dissous (après adjonction du liquide aqueux à tester mais sans adjonction de nutrient) est comprise entre 50 et 100 % de la saturation du liquide en gaz oxygéné considéré, de préférence entre 70 et 95 % de cette même saturation.

La nature et la quantité de nutrient ajouté (après soutirage d'une fraction de culture bactérienne mais avant le réacteur piston) sont choisis habituellement de manière telle que la teneur en gaz oxygéné dissous du mélange à la sortie du réacteur piston (en absence de toxicité) soit comprise entre 0 et 95 %, de préférence entre 30 et 85 % de la saturation du liquide à tester en gaz oxygéné.

Dans le but de parfaire la fiabilité et le reproductibilité des procédés et appareils selon l'invention on effectue avantageusement toutes les opérations à température constante ce qui conduit à thermostater aussi bien la culture bactérienne que le liquide aqueux à tester, le nutrient, le réacteur piston et la cellule de mesure de l'oxygène dissous.

Lorsque le procédé de l'invention fonctionne de façon continue on opère généralement en respectant les caractéristiques suivantes :

. le nombre de bactéries vivantes par cm3 de suspension bactérienne (c'est-à-dire de la fraction de culture bactérienne destinée à être mélangée avec le liquide aqueux à tester ; le nombre de bactéries vivantes indiqué étant valable avant le mélange de cette fraction avec ce liquide aqueux à tester) est compris entre $2 \times 10^6$ et $2 \times 10^{10}$, de préférence comprise entre $5 \times 10^8$ et $1 \times 10^{10}$.

. le rapport $\dfrac{V_1}{V_2}$ ($V_1$ et $V_2$ étant les volumes par unité de temps respectivement du liquide aqueux à tester et de la fraction de culture bactérienne dont le mélange est introduit à l'entrée du réacteur piston) est généralement compris entre 1 et 40, de préférence entre 1,5 et 20.

Outre le procédé de l'invention sus décrit, l'invention porte encore sur un appareillage permettant de réaliser le dit procédé.

.../...

Cet appareillage est constitué essentiellement :

a) d'un réacteur biologique permettant d'assurer une culture bactérienne aérobie stable et en suspension aqueuse.

b) d'un réacteur piston,

c) d'une cellule de mesure de gaz oxygéné dissous dans le liquide aqueux sortant du réacteur piston et contenu dans cette cellule, ladite cellule étant pourvue de moyens de mesure de la teneur en oxygène dissous.

d) de moyens de soutirage d'une fraction de la culture bactérienne contenue dans le réacteur a) et d'alimentation de cette fraction dans le réacteur piston b)

e) de moyens d'alimentation de réacteur piston en nutrient

f) de moyens d'alimentation du réacteur piston en liquide aqueux à tester

g) de moyens d'alimentation du réacteur piston en agent nettoyant

h) de moyens de soutirage du liquide contenu dans la cellule de mesure c).

Chacun des éléments a) et g) peut être thermostaté.

Le réacteur biologique a) est généralement constitué d'un simple récipient, de préférence thermostaté, pourvu de moyens d'alimentation en gaz oxygéné (pouvant être sous forme d'air) et en agents nutritifs.

Le réacteur piston b) est tel qu'il a déjà été défini à propos du procédé selon l'invention.

La cellule de mesure c) est généralement constituée d'un petit récipient pourvu d'une entrée, d'une sortie et d'une sonde à oxygène plongeant dans ledit récipient, l'entrée de cette cellule étant reliée à la sortie du réacteur piston. Des caractéristiques de cette cellule ont déjà été développées plus haut. Les moyens d'alimentation de la cellule de mesure sont tout simplement, en pratique, la sortie du réacteur piston.

Les moyens de soutirage et d'alimentation d) sont le plus souvent constitués d'un tube. Il en est de même des moyens d'alimentation e), f) et g) ; selon une modalité avantageuse les moyens d), e), f) et g) sont réunis pour constituer une association de 4 tubes convergeant pour en former un seul.

Les moyens de soutirage h) sont aussi constitués le plus souvent d'un tube.

Ces divers éléments et notamment les moyens d'alimentation et de soutirage peuvent être compléter par des vannes ou robinets permettant de régler les débits, ainsi que par divers appareils, notamment des débitmètres ou des thermomètres.                    .../...

Enfin, il est évident que la sonde à oxygène est de préférence reliée à un appareil enregistreur qui permet d'inscrire directement la teneur en oxygène sous forme de graphique, la présence d'agents toxiques apparaissant dès lors sous forme de pic dans le graphique.

L'invention concerne encore un autre procédé et l'appareillage correspondant, lesquels constituent chacun un perfectionnement du procédé et de l'appareillage déjà décrits.

Selon ce second procédé, au lieu de procéder à mesure unique de la teneur en oxygène du seul mélange culture bactérienne + liquide aqueux à tester, on effectue une double mesure ou une mesure différentielle, c'est-à-dire qu'on mesure simultanément ou différentiellement la teneur en oxygène dissous d'un mélange culture bactérienne + liquide aqueux à tester et d'un autre mélange culture bactérienne + eau (ce deuxième mélange est ainsi un mélange "de référence"), ces 2 mélanges ayant été obtenus et traités de manière rigoureusement identique, notamment en ce qui concerne les débits, le réacteur piston, l'adjonction de nutrient et la cellule de mesure ; la même culture bactérienne alimente les deux mélanges. Autrement dit, ce procédé, de préférence continu, consiste à procéder à une culture bactérienne de manière à avoir une suspension aqueuse à caractéristiques stables, à introduire séparément deux fractions de cette culture dans deux réacteurs piston distincts, à introduire dans ces deux réacteurs simultanément avec ces deux fractions d'une part de l'eau pour un réacteur et d'autre part le liquide aqueux à tester pour l'autre réacteur, à mesurer soit les teneurs en gaz oxygéné dissous à la sortie des deux réacteurs, soit la différence des teneurs en gaz oxygéné dissous à la sortie des deux réacteurs. Il est préférable, en raison du contrôle par l'agent nettoyant, de ne pas mesurer directement cette différence de teneur mais de procéder séparément à la mesure des teneurs en gaz oxygéné dans les deux cellules et à comparer les 2 mesures (par exemple par comparaison de visu des enregistrements).

Toutes les autres caractéristiques subsidiaires du premier procédé (culture bactérienne, débits relatifs, adjonction de nutrient, saturation en air ou oxygène, teneurs en oxygène dissous etc...) s'appliquent mutatis mutandis au second procédé perfectionné. L'eau utilisée comme liquide de référence peut être de l'eau pure ou plus simplement de l'eau ordinaire telle que l'eau appelée eau du robinet (en anglais :

.../...

"tap water"), c'est -à-dire de l'eau contenant des éléments inertes vis à vis de la culture bactérienne.

Le second appareillage selon l'invention permettant de mettre en oeuvre le second procédé selon l'invention comprend donc :

a) un réacteur biologique permettant d'assurer une culture bactérienne aérobie stable et en suspension aqueuse

b) un premier réacteur piston

b') un second réacteur piston

c et c') deux cellules de mesure de gaz oxygéné dissous dans le liquide aqueux contenu dans ces cellules et sortant respectivement des réacteurs piston b et b', les dites cellules étant pourvues de moyens de mesure de la teneur en oxygène dissous.

d) des moyens de soutirage d'une fraction de la culture bactérienne contenu dans le réacteur a) et d'alimentation de cette fraction dans le premier réacteur piston b).

d') des moyens de soutirage d'une fraction de la culture bactérienne contenue dans le réacteur a) et d'alimentation de cette fraction dans le second réacteur piston b')

e et e') des moyens d'alimentation respectivement des réacteurs piston b et b' en nutrient.

f) des moyens d'alimentation du réacteur piston b') en eau

g et g') des moyens d'alimentation du réacteur piston en agent nettoyant

h et h') des moyens de soutirage des liquides contenus dans les cellules de mesure c et c'.

Les particularités des éléments a) à h') sont mutatis mutandis celles définies pour les éléments a) à h) du premier appareillage selon l'invention.

Dans la figure 1 on décrit un premier appareillage selon l'invention permettant la réalisation du premier procédé selon l'invention.

Dans la figure 2 on décrit un second appareillage selon l'invention permettant la réalisation du second procédé selon l'invention.

Les pompes éventuellement nécessaire pour assurer l'écoulement de tel ou tel fluide ou même pour réguler le débit de tel ou tel fluide n'ont pas été représentées. Il est évident qu'elles sont disposées et utilisées de manière à respecter les caractéristiques des procédés de l'invention telles que définies dans la description ci-dessus.

.../...

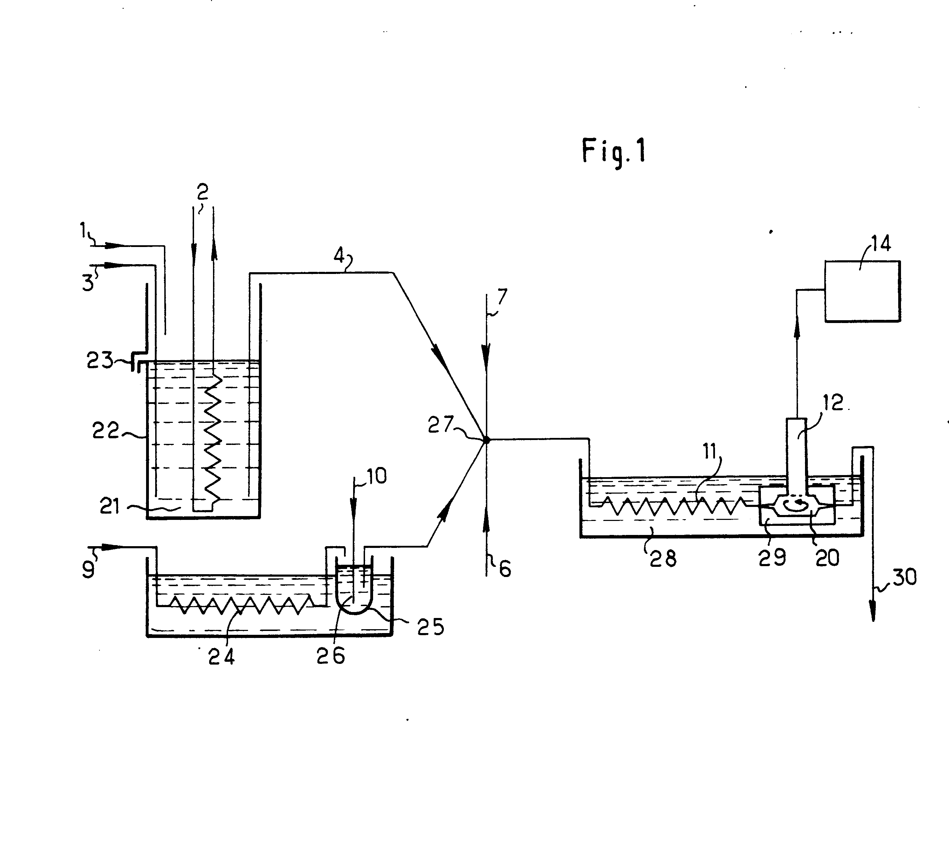

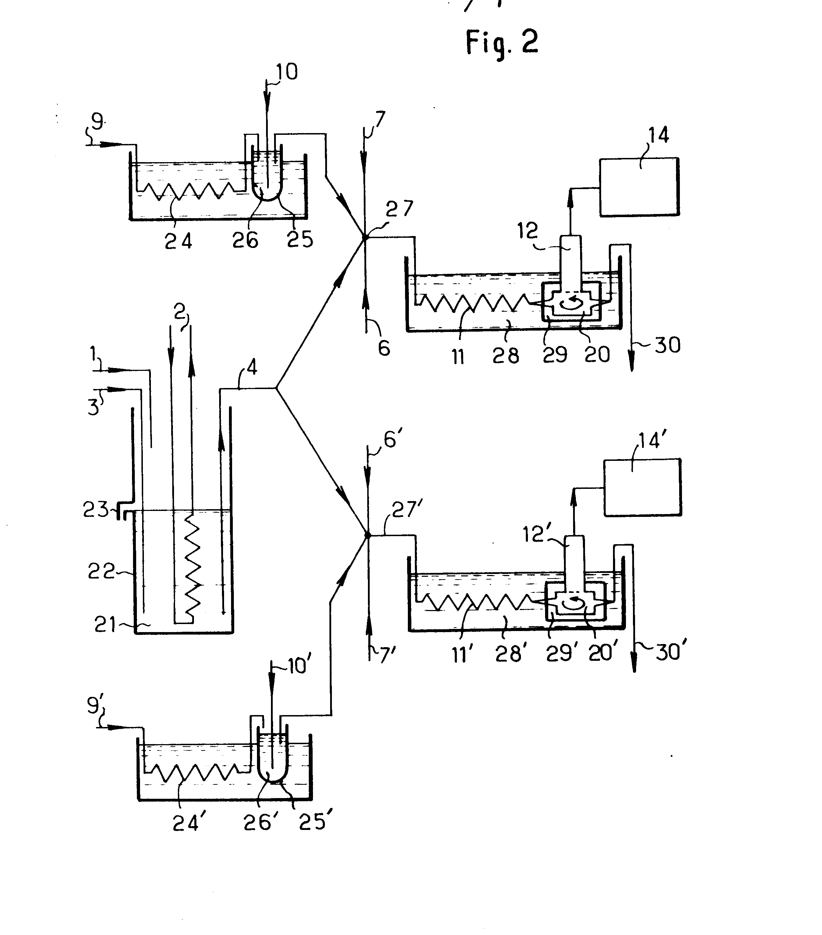

Il doit bien être entendu que les appareillages des figures 1 et 2 ne sont donnés qu'à titre illustratif non limitatif et que l'invention ne saurait être restreinte aux aspects particuliers ressortant de ces figures.

Une culture bactérienne (21) est obtenue dans un réacteur biologique (22) constitué d'un récipient pourvu d'un trop-plein (23), d'un système (3) d'alimentation en gaz oxygéné, d'une alimentation (1) en substrat nutritif des bactéries, de moyens (2) de thermostatation et d'une canalisation (4) de soutirage.

Le liquide aqueux à tester est introduit en (9) ; il est thermostaté par le système (24) de thermostatation (en l'occurence un serpentin noyé dans un bain liquide à température constante) ; puis il est saturé de gaz oxygéné dans le dispositif (25) ; en l'occurence le dispositif (25) est un système à barbotage, le gaz oxygéné étant introduit en (10) et barbotant dans le liquide aqueux thermostaté (26).

Ce liquide ainsi traité est mélangé en (27) avec la culture bactérienne venant de la canalisation (4), le nutrient venant de la canalisation (6) et, de temps en temps, avec l'agent nettoyant venant par la canalisation (7). Le mélange ainsi réalisé est introduit dans le réacteur piston (11) thermostaté par le bain (28). En l'occurence ce réacteur piston est un tube en serpentin (de verre ou de préférence en téflon ou autre matière polymérique lisse et inerte). A la sortie du réacteur piston le liquide entre dans la cellule de mesure (29) pourvue d'une agitation (20) (en l'occurence il s'agit d'une agitation par barreau magnétique). La sonde à oxygène (12) (en l'ocurrence une électrode à oxygène) transmet au système (14) d'amplification / enregistrement l'indication de la teneur en gaz oxygéné dissous du liquide dans la cellule de mesure (29). La canalisation (30) assure l'élimination du contenu de la cellule (29).

Dans la figure 2, les symboles ont la même signification. Les éléments désignés par les chiffres 6', 7', 9', 11', 12', 14', 20', 24', 25', 26', 27', 28', 29', 30' sont semblable à ceux désignés par les chiffres 6, 7, 9, 11, 12, 14, 20, 24, 25, 26, 27, 28, 29, 30, la seule différence étant qu'en (9') on introduit de l'eau simple au lieu du liquide aqueux à tester.

En (14) et (14') on peut soit procéder à deux enregistrements simultanés et, de visu, à les comparer, soit réunir les deux signaux issus des sondes (12) et (12') et enregistrer la différence.

.../...

Les appareillages de l'invention peuvent évidemment être complétés par les dispositifs les plus divers notamment par des systèmes d'alarme (en cas de présence de toxicité) ou par des systèmes d'asservissement et/ou télécommande permettant d'arrêter l'écoulement du liquide aqueux dont on a repéré la toxicité.

Le procédé de l'invention est particulièrement avantageux en raison des signaux de toxicité nets, précis, clairs et importants qu'il permet d'obtenir.

Les exemples suivants sont également donnés à titre non limitatif et ils illustrent des modes particuliers de réalisation de l'invention.

EXEMPLE 1

Un dispositif selon la figure 1 est utilisé.

Le réacteur biologique (21) contient 800 cm3 d'une suspension bactérienne comportant $5.10^9$ bactéries vivantes/cm3 et thermostatée à 35°C. Cette suspension, ensemencée initialement à l'aide d'une entérobactérie du genre "Citrobacter" est entretenue de la manière suivante :

. aération par introduction en (3) de 120 l/h d'air

. alimentation en (1) d'un mélange de 3 liquides :

- Le 1er liquide alimenté à raison de 300 cm3/h est de l'eau potable de ville appelée "eau du robinet" (tap water en anglais) traitée préalablement avec du charbon actif.

- Le 2ème liquide alimenté à raison de 10 cm3/h est une solution aqueuse qui contient :

. 40 g/l de glucose

. 5 g/l d'extrait de levure

. 5 g/l de sulfate de magnésium heptahydraté

. 1 g/l du sel double trihydraté de $Na^+$ et de $Fe^{+++}$ de l'acide éthylène dinitrilotétraacétique (appelé également EDTA ferrique)

. 0,1 g/l de $MnCl_2$, $4H_2O$

. 9,5 g/l de solution aqueuse à 85 % en poids de $H_3PO_4$

. 5 g/l de solution aqueuse à 96 % en poids de $H_2SO_4$

. 10 cm3/l d'une solution aqueuse d'oligo éléments contenant 0,3 mg de sulfate de cuivre, 0,6 mg de molybdate d'ammonium, 0,6 mg de sulfate de zinc et 0,6 mg de chlorure de cobalt pour 10 cm3 d'eau.

- Le 3ème liquide alimenté à raison de 10 cm3/h est une

.../...

solution aqueuse qui contient 30 g/l d'acide maléique.

30 g/l de NaOH

30 g/l de tampon Tris (tris(hydroxyméthyl) aminométhane).

Parallèlement le liquide aqueux à tester est introduit en (9) sa température est réglée à 20°C dans le dispositif (24) tandis que le système à barbotage (25) permet de le saturer en air.

En (27) arrive simultanément un débit de 250 cm3/h de culture bactérienne par la canalisation (4), un débit de 1200 cm3/h de liquide aqueux à tester, un débit de 16 cm3/h de nutrient par la canalisation (6). Ce nutrient est une solution aqueuse contenant 10 g/l de glucose et 1 millimole/l d'acide sulfurique.

Par la canalisation (7), on introduit de l'acide formique (agent nettoyant) à raison de 20 cm3 /h (débit instantané) pendant des périodes de durée 7 mn espacées entre elles par des intervalles de 2 h.

Le réacteur piston est un tube de 20 m de long et 2 mm de diamètre interne ; il est en téflon (= polytétrafluoroéthylène) ; il est enroulé en hélice dans le but de gagner de la place et de réduire les dimensions du bain thermostaté (28) ; thermostatation à 20°C.

La cellule de mesure (29) a un volume interne de 2 cm3 ; la sonde à oxygène (12) est une sonde polarographique pourvue d'une membrane en téflon perméable à l'oxygène et placée au contact du liquide dans la cellule.

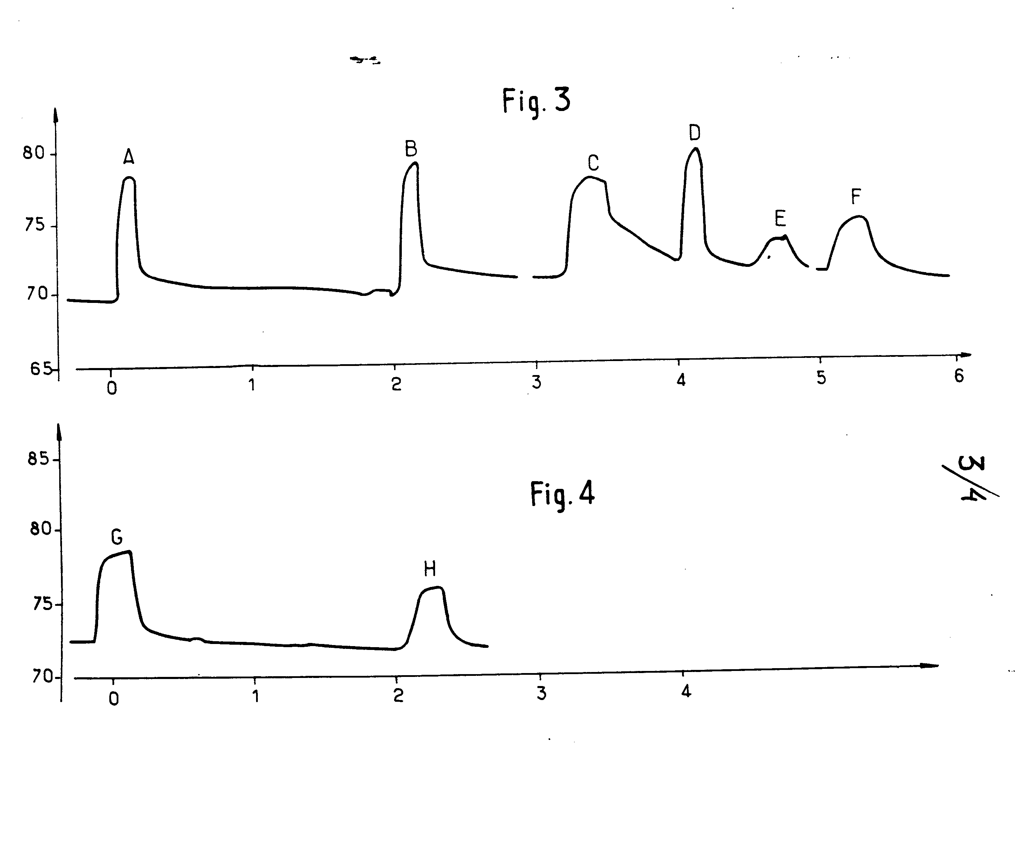

On obtient ainsi dans l'appareil d'enregistrement un graphique qui a été représenté à la figure 3. Sur ce graphique, le temps est porté en abscisse et en ordonnée le pourcentage de la saturation en air à 20°C du mélange liquide sur lequel la mesure est faite.

La ligne de base est stable et stabilisée à une teneur en air dissous équivalent à environ 70 % de la saturation en air.

L'introduction périodique de l'acide formique donne naissance aux pics étroits et réguliers représentés en A, B et D ; l'apparition de ces pics a lieu 2 minutes et 30 secondes après le début de l'injection d'acide formique. Le signal de toxicité produit par l'appareil et le procédé de l'invention est ainsi particulièrement rapide, clair, net et non ambigu.

On a aussi procédé à des injections de $Cu^{++}$ dans le liquide à tester pendant des durées de 15 mn chaque fois. Le pic C a été obtenu

...../...

a été obtenu avec la présence de 50 mg/l de $Cu^{+++}$ dans le liquide aqueux à tester ; le pic E a été obtenu avec la présence de 1 mg/l de $Cu^{++}$ dans le liquide aqueux à tester ; le pic F a été obtenu avec la présence de 3,5 mg/l de $Cu^{++}$ dans le liquide aqueux à tester.

On a ensuite poursuivi l'expérimentation sans injection périodique d'acide formique mais avec injections de $Cd^{++}$ pendant des durées de 15 mn chaque fois.

Le graphique d'enregistrement obtenu est représenté à la figure 4 où le pic G correspond à la présence de 35 mg/l de $Cd^{++}$ dans le liquide aqueux à tester et où le pic H correspond à la présence de 10 mg/l de $Cd^{++}$.

EXEMPLE 2

Un dispositif selon la figure 1 est utilisé.

Le réacteur biologique (21) contient 800 cm3 d'une suspension bactérienne comportant $7 \times 10^9$ bactéries vivantes/cm3 et thermostatée à 35°C.

Cette culture bactérienne se distingue de celle utilisée à l'exemple 1 simplement par les points suivants :

- le 2ème liquide est alimenté à 7,5 cm3/h (au lieu de 10) et contient 60 g/l de glucose (au lieu de 40) et 20 g/l d'extrait de levure (au lieu de 5).

- le 3ème liquide est alimenté à un débit permettant le maintien du pH du mélange à une valeur de 7,05.

Le liquide aqueux à tester est introduit en (9) ; sa température est réglée à 25°C dans le dispositif (24) tandis que le système à barbotage (25) permet de le saturer en air.

En (27) arrive simultanément un débit de 300 cm3/h de culture bactérienne par la canalisation (4), un débit de 1200 cm3/h de liquide aqueux à tester, un débit de 16 cm3/h de nutrient par la canalisation (6). Ce nutrient est une solution aqueuse contenant 3,5 g/l de glucose, 1 g/l d'extrait de levure et 200 g/l de NaCl.

Par la canalisation (7) on introduit l'agent nettoyant qui est constitué par de l'acide formique pur. Il est alimenté à raison de 20 cm3/h pendant des périodes de 15 secondes chacune, séparées entre elles par un intervalle de 30 minutes.

Le réacteur piston est un tube de 20 m de long dont le diamètre interne, la constitution, la disposition et la température sont

.../...

semblables à ceux de l'exemple 1.

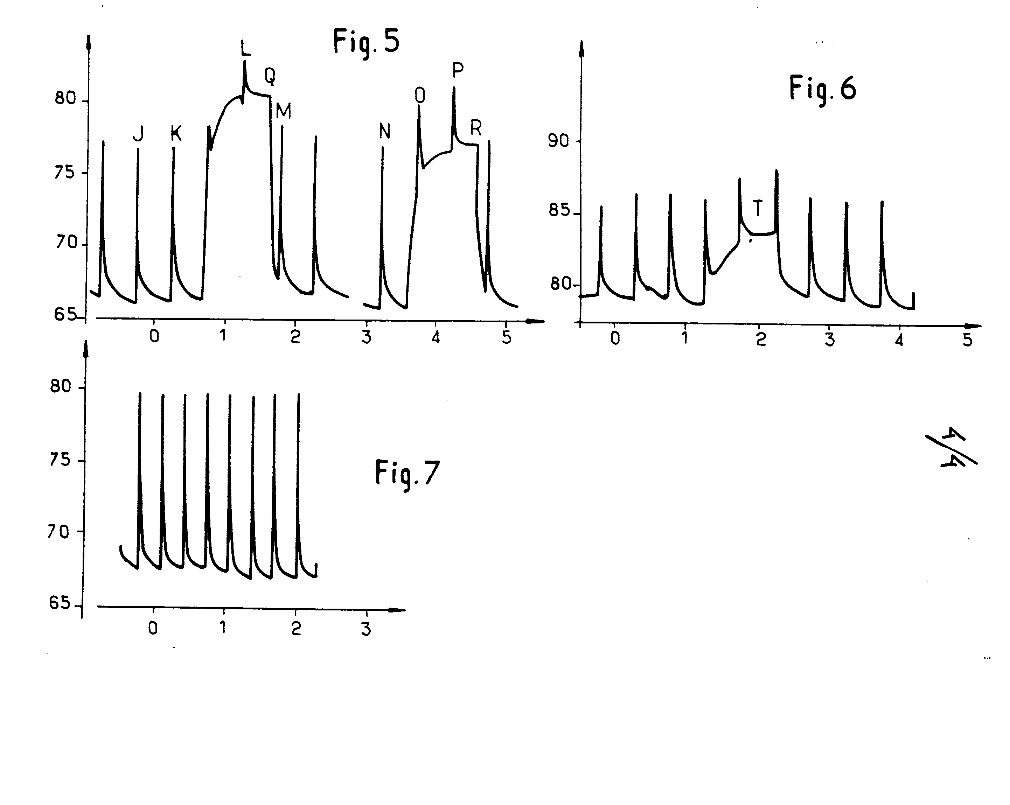

On obtient ainsi en (14) un enregistrement du signal de toxicité qui a été représenté à la figure 5.

La ligne de base est stabilisée à environ 66 % de la saturation en air à 25°C.

L'introduction périodique de l'acide formique donne naissance aux pics étroits, réguliers et pointus représentés notamment en J, K, L, M, N, O, P ; l'apparition de ces pics a lieu 2 minutes et 10 secondes après le début de l'injection d'acide formique.

Le passage de $Hg^{++}$ dans le liquide aqueux à tester pendant 1 heure à raison de 1 mg/1 provoque un pic très net et très marqué qui culmine en Q.

La suppression de l'alimentation en (6) du nutrient conduit à un déplacement de la ligne de base qui se stabilise en R à 77 % de la saturation en air.

EXEMPLE 3

On reproduit l'exemple 2 avec comme seule modification, le fait que le débit de culture bactérienne alimentée en (4) est de 150 $cm^3$/h (au lieu de 300).

On obtient le graphique d'enregistrement représenté à la figure 6.

La ligne de base est stabilisée à 79 % de la saturation en air à 25°C.

L'injection périodique d'acide formique comme à l'exemple 2 donne naissance à des pics pointus, étroits et réguliers apparaissant 2 minutes 25 secondes après le début de l'injection.

Le passage de $Hg^{++}$ dans le liquide aqueux à tester à raison de 0,35 mg/1 pendant 1 heure provoque un pic net et massif culminant en T.

EXEMPLE 4

Un dispositif selon la figure 1 est utilisé.

Le réacteur biologique (21) contient 800 cm3 d'une suspension bactérienne comportant 5 x $10^9$ bactéries vivantes/cm3 et thermostaté à 35°C.

Cette suspension, ensemencée initialement par un mélange d'entérobactéries dont la bactérie dominante est du genre "Citrobacter", est entretenue de la manière suivante :

.../...

- aération par introduction en (3) de 120 1/h d'air.

- alimentation en (1) d'un mélange de 3 liquides

. le 1er liquide est le même en nature et en débit que son homologue de l'exemple 1.

. le 2ème liquide alimenté à raison de 7,5 cm3/h est une solution aqueuse qui contient.

. 120 g/1 de glucose

. 10 g/1 de sulfate d'ammonium

. 40 g/1 d'extrait de levure

. 10 g/1 de $MgSO_4$, $7H_2O$

. 2 g/1 d'EDTA ferrique

. 0,2 g/1 de Mn $Cl_2$, $4H_2O$

. 6 g/1 de $KH_2PO_4$

. 200 g/1 de NaCl

. 10 cm3/1 de la solution aqueuse d'oligo-éléments utilisée à l'exemple 1.

. le 3ème liquide est de même nature que sont homologue de l'exemple 1 et il est alimenté à un débit permettant de maintenir le pH à 7,05.

Parallèlement, le liquide aqueux à tester est introduit en (9) ; sa température est réglée à 20°C dans le dispositif (24) tandis que le système à barbotage (25) permet de le saturer en air.

En (27) arrive simultanément un débit de 300 cm3/h de culture bactérienne par la canalisation (4), un débit de 1200 cm3/h de liquide aqueux à tester, un débit de 16 cm3/h de nutrient par la canalisation (6). Ce nutrient est le même que celui utilisé à l'exemple 1.

Par la canalisation (7), on introduit l'agent nettoyant qui est de l'acide formique. Il est alimenté à raison de 20 cm3/h (débit instantané) pendant des périodes de durée de 15 secondes, deux injections successives étant séparée par un intervalle de 20 minutes.

Le réacteur piston a 11 m de long, ses autres caractéristiques étant semblables à celles du réacteur piston de l'exemple 1.

On obtient ainsi en (14) un graphique d'enregistrement qui a été représenté à la figure 7. Les pics obtenus sont tout à fait remarquables par leur étroitesse et leur amplitude. La ligne de base est stabilisée à 68 % de la saturation en air à 25°C. L'apparition des pics sur l'enregistrement a lieu 1 minute 30 secondes après l'injection d'acide ferrique.

.../...

## REVENDICATIONS

1) - Procédé de détection et/ou de mesure de toxicité de liquides aqueux caractérisé en ce que l'on procède à une culture de bactéries aérobies de manière à avoir une suspension aqueuse bactérienne à caractéristiques stables, qu'une fraction de cette culture de bactéries est mélangée au liquide aqueux à tester préalablement saturé en gaz oxygéné et le mélange est introduit dans un réacteur piston, puis l'on mesure la quantité d'oxygène présent dans le liquide à la sortie de ce réacteur piston.

2) - Procédé selon la revendication 1 caractérisé en ce que le réacteur piston est un tube de surface interne lisse et dont le rapport longueur/diamètre est supérieur à 300, de préférence compris entre 5000 et 50 000.

3) - Procédé selon l'une des revendications 1 ou 2 caractérisé en ce que le temps de séjour du mélange dans le tube est compris entre 30 secondes et 15 minutes, de préférence entre 1 et 5 minutes.

4) - Procédé selon l'une des revendications 1 à 3 caractérisé en ce qu'on injecte périodiquement dans le réacteur piston un agent nettoyant, de préférence biocide et acide.

5) - Procédé selon l'une des revendications 1 à 4 caractérisé en ce que la mesure de la quantité d'oxygène se fait à l'aide d'une sonde à oxygène dont la cellule de mesure où se trouve le mélange sur lequel on fait la mesure a un volume compris entre 0,05 et 10 cm3, de préférence inférieur à 5 cm3.

6) - Procédé selon l'une des revendications 1 à 5 caractérisé en ce que le mélange (culture bactérienne) + (liquide aqueux à tester) à l'entrée du réacteur piston est complété par un nutrient.

7) - Procédé selon l'une des revendications 1 à 6 caractérisé en ce que la teneur en gaz oxygéné du mélange (culture bactérienne) + (liquide aqueux à tester) sans nutrient est comprise entre 50 et 100 % de la saturation en gaz oxygéné, de préférence entre 70 et 95 %, et que la teneur en oxygène dissous du mélange à la sortie du réacteur piston (en l'absence de toxicité) est comprise entre 0 et 95 % de

.../...

la saturation en gaz oxygéné, de préférence entre 30 et 85 %.

8) - Appareillage, utilisable notamment pour la détection et/ou la mesure de la toxicité de liquides caractérisé en ce qu'il comprend.

a) un réacteur biologique permettant d'assurer une culture bactérienne-aérobie stable et en suspension aqueuse.

b) un réacteur piston.

c) une cellule de mesure d'oxygène dissous dans le liquide aqueux sortant du réacteur piston et contenu dans cette cellule,

d) des moyens de soutirage d'une fraction de la culture bactérienne contenu dans le réacteur a) et d'alimentation de cette fraction dans le réacteur piston b).

e) des moyens d'alimentation du réacteur piston en nutrient

f) des moyens d'alimentation du réacteur piston en liquide aqueux à tester.

g) de moyens d'alimentation du réacteur piston en agent nettoyant.

h) des moyens de soutirage du liquide contenu dans la cellule de mesure c).

9) - Appareillage, utilisable notamment pour la détection et/ou la mesure de la toxicité de liquides caractérisé en ce qu'il comprend :

a) un réacteur biologique permettant d'assurer une culture bactérienne aérobie stable et en suspension aqueuse.

b) un premier réacteur piston

b') un second réacteur piston

c et c') deux cellules de mesure d'oxygène dissous dans le liquide aqueux contenu dans ces cellules et sortant respectivement des réacteurs piston b et b'.

d et d') des moyens de soutirage de fractions de la culture bactérienne contenue dans le réacteur a) et d'alimentation de ces fractions respectivement dans les réacteurs piston b et b'

e et e') des moyens d'alimentation respectivement des réacteurs piston b et b' en nutrient.

f) des moyens d'alimentation du réacteur piston b en liquide aqueux à tester.

f') des moyens d'alimentation du réacteur piston b' en eau.

...../...

g et g') des moyens d'alimentation du réacteur piston en agent nettoyant

h et h') des moyens de soutirage des liquides contenues dans les cellules de mesure c et c'.

1/4

Fig.1

Fig. 3

Fig. 4

3/4

0006376

Fig.5

Fig.6

Fig.7

4/4

0006376

# 0006376

Office européen
des brevets

**RAPPORT DE RECHERCHE EUROPEENNE**

Numéro de la demande

EP 79 400 360.8

| | DOCUMENTS CONSIDERES COMME PERTINENTS | | CLASSEMENT DE LA DEMANDE (Int. Cl.3) |
|---|---|---|---|
| Catégorie | Citation du document avec indication, en cas de besoin, des parties pertinentes | Revendication concernée | |
| | DE – A – 2 634 846 (BAYER AG.) <br> * en entier * | 1,4, <br> 8,9 | G 01 N 33/18 <br> C 02 F 3/00 |
| | --- | | |
| | US – A – 4 073 692 (CIACCIO et al.) <br> * revendication 14 et colonne 7, <br> lignes 15 à 30 * | 1,2 <br> 8 | |
| | -- | | DOMAINES TECHNIQUES RECHERCHES (Int. Cl.²) |
| | DE – A – 2 513 650 (BROWN, BOVERI <br> & CIE) <br> * revendications 1, 2 * | 1 | C 02 C 5/00 <br> G 01 N 33/18 |
| | -- | | |
| | FR – A – 2 321 128 (STAMICARBON) <br> * revendications 1 à 4 * | 1 | |
| | -- | | |
| A | DE – A – 2 651 896 (BROWN, BOVERI <br> & CIE) <br> * en entier * | | |
| | ---- | | |

CATEGORIE DES DOCUMENTS CITES

X: particulierement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille. document correspondant

| Le present rapport de recherche a ete etabli pour toutes les revendications | | |
|---|---|---|
| Lieu de la recherche <br> Berlin | Date d achevement de la recherche <br> 20-08-1979 | Examinateur <br> SCHWARTZ |

OEB Form 1503.1 06.78